# DEMANDE DE BREVET EUROPEEN

(11) **EP 3 628 389 A1**
(43) Date de publication de la demande: **01.04.2020**
(21) Numéro de dépôt: 19306177.7
(22) Date de dépôt: 24.09.2019
(51) Int. Cl.: B01D 53/00, B01D 53/75, C10L 3/10, F25J 1/00

(54) **PROCÉDÉ DE PRODUCTION DE BIOMÉTHANE À PARTIR D'UN FLUX DE BIOGAZ COMPRENANT UNE SOLIDIFICATION DES IMPURETÉS**

(30) Priorité: 25.09.2018 FR 1858702
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: CARDON, Guillaume, 78350 Les Loges-En-Josas (FR); TRUEBA, Antonio, 78350 Les Loges-En-Josas (FR); VALENTIN, Solène, 78350 Les Loges-En-Josas (FR)
(74) Mandataire: Laigneau, Amandine

(57) **Abrégé**

Procédé de production de biométhane à partir d'un flux de biogaz comprenant du méthane, du dioxyde de carbone et au moins une impureté choisie parmi l'ammoniac, les composés organiques volatils, l'eau, les impuretés soufrées (H₂S) et les siloxanes, comprenant :
- une première étape de séchage du flux de biogaz,
- une deuxième étape d'élimination au moins partiel de ladite impureté contenue dans le flux de biogaz séché par solidification et retrait de l'impureté ; et
- une troisième étape de séparation du méthane et du dioxyde de carbone contenu dans le biogaz issu de la deuxième étape de manière à produire un flux de biométhane et un flux de CO₂.

## Description

La présente invention est relative à un procédé de production de biométhane à partir d'un flux de biogaz comprenant du méthane, du dioxyde de carbone et au moins une impureté choisie parmi l'ammoniac, les composés organiques volatils, l'eau, les impuretés soufrées (H2S) et les siloxanes .

Elle concerne en particulier l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.

Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié.

La présente invention se propose de fournir un procédé amélioré de production de biométhane.

Une solution de la présente invention est un procédé de production de biométhane à partir d'un flux de biogaz comprenant du méthane, du dioxyde de carbone et au moins une impureté choisie parmi l'ammoniac, les composés organiques volatils, l'eau, les impuretés soufrées (H₂S) et les siloxanes, comprenant :
- une première étape de séchage du flux de biogaz,
- une deuxième étape d'élimination au moins partiel de ladite impureté contenue dans le flux de biogaz séché par solidification et retrait de l'impureté ; et
- une troisième étape de séparation du méthane et du dioxyde de carbone contenu dans le biogaz issu de la deuxième étape de manière à produire un flux de biométhane et un flux de CO2.

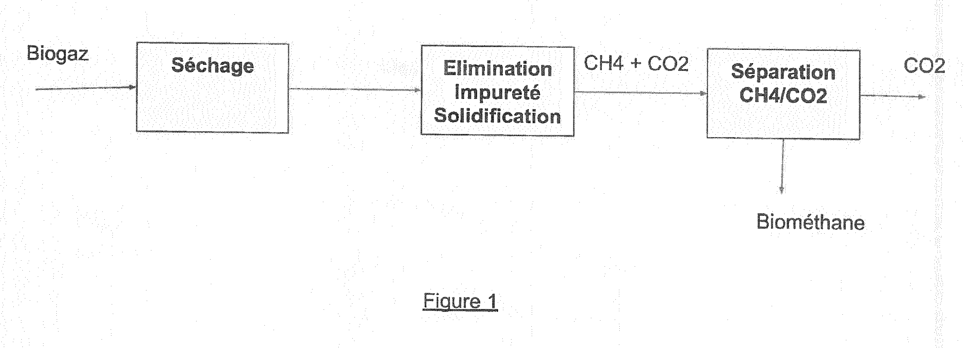

Ces trois étapes sont illustrées par la figure 1.

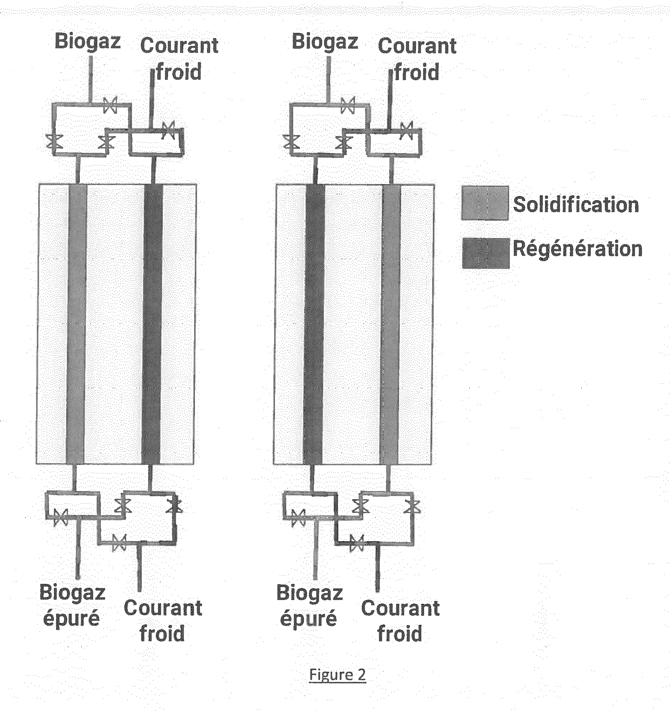

Selon le cas, le procédé selon l'invention peut présenter une ou plusieurs des caractéristiques suivantes :
- la deuxième étape d'élimination au moins partiel de ladite impureté comprend une première sous-étape de compression du flux de biogaz séché à une pression supérieure à 5 bar, de préférence 10 bar, une deuxième sous-étape de solidification de ladite impureté par refroidissement du flux de biogaz comprimé à une température inférieure à -70°C ; et une troisième sous-étape de dépôt et de retrait de ladite impureté. De préférence cette deuxième étape est cyclique de manière à maintenir le procédé en fonctionnement continu ;
- à la deuxième sous-étape le flux de biogaz est refroidi par un flux de refroidissement, de préférence un flux de CO₂, produit de préférence lors de la troisième étape.
- dans la troisième sous-étape ladite impureté est retirée par sublimation de ladite impureté et balayage d'un flux gazeux de balayage.
- la sublimation est effectuée à une température supérieure à la température de sublimation et une pression inférieure à 2 bar.
- à la deuxième sous-étape le flux de biogaz est refroidi par un flux de refroidissement et à la troisième sous-étape le flux de refroidissement issu de la deuxième sous-étape est utilisé comme flux gazeux de balayage.
- de préférence la deuxième sous-étape et la troisième sous-étape sont réalisées simultanément en une seule et même sous-étape ;
- le flux gazeux de balayage est un flux de CO₂ ou d'azote.
- le flux gazeux de balayage est issu d'une capacité de stockage.
- le flux gazeux de balayage est le recycle du flux de CO₂ produit lors troisième étape de séparation du méthane et du dioxyde de carbone contenu dans le biogaz
- la troisième étape de séparation du méthane et du dioxyde de carbone contenu dans le biogaz est effectuée par lavage à l'eau ou aux amines, adsorption, solidification du dioxyde de carbone ou séparation membranaire.
- la première étape de séchage du flux de biogaz est effectuée par refroidissement et condensation puis adsorption ;
- la deuxième étape d'élimination au moins partiel de ladite impureté met en œuvre un échangeur de chaleur.
- la première sous-étape de compression du flux de biogaz est effectuée à l'aide d'un compresseur.
- la deuxième étape d'élimination au moins partiel de ladite impureté met en œuvre un échangeur de chaleur, à la deuxième sous-étape le flux de biogaz est refroidi par un flux de refroidissement avec le flux de biogaz passant à travers un premier canal de l'échangeur de chaleur et le flux de refroidissement passant à travers un deuxième canal de l'échangeur de chaleur, et à la troisième sous-étape ladite impureté est retirée par sublimation de ladite impureté et balayage d'un flux gazeux de balayage avec le flux gazeux de balayage passant dans le premier canal de l'échangeur de chaleur; avec le flux de biogaz passant dans le deuxième canal pendant la troisième sous-étape. Notons que le premier canal est ainsi régénéré et que la deuxième étape peut à nouveau être mise en œuvre de manière cyclique. Notons que de préférence le flux de balayage est un flux de CO₂ et le flux de refroidissement également un flux de CO₂. Dans ce cas préférentiel, le flux de CO₂ passe dans le deuxième canal puis dans le premier canal par l'intermédiaire d'un jeu de vannes. De la même manière le flux de biogaz passe dans le premier canal puis dans le deuxième canal par l'intermédiaire d'un jeu de vannes. La figure 2 illustre ce cas préférentiel.

La première étape de séchage est nécessaire pour éliminer toutes traces d'eau du flux de biogaz. En effet, l'eau présente dans le flux de biogaz pourrait se condenser dans le compresseur et l'endommager.

La deuxième étape d'élimination de ladite impureté est de préférence un processus cyclique qui fonctionne en 3 sous-étapes :
- compression du flux de biogaz à une pression supérieure à 5 bar, de préférence 10 bar
- solidification dans un échangeur de chaleur des impuretés comprises dans le biogaz par refroidissement. On observe ainsi un dépôt des impuretés dans l'échangeur de chaleur. Et en sortie de l'échangeur, du biogaz purifié est récupéré.
- sublimation des impuretés qui se sont déposées dans l'échangeur de chaleur de manière à régénérer l'échangeur de chaleur. La sublimation des impuretés se fait à basse pression, c'est-à-dire à une pression inférieure à 2 bar. De préférence un flux de CO₂ est injecté dans l'échangeur de chaleur afin de balayer les impuretés passées en phase gazeuse. Le CO₂ permet par la même occasion le maintien en froid de l'ensemble flux de biogaz - flux de CO₂.

Le CO₂ peut provenir d'une capacité de stockage ou du biogaz lui-même. Dans ce dernier cas il correspondrait à tout ou partie du flux de CO₂ produit à la troisième étape du procédé selon l'invention. Notons que le CO₂ peut être remplacé par un autre gaz tel que l'azote....

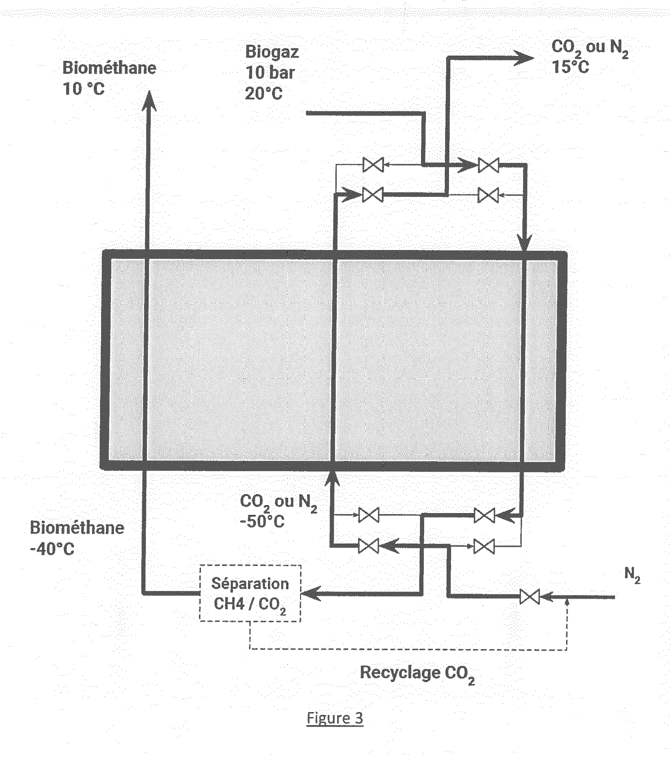

En sortie de l'échangeur de chaleur, le biogaz purifié peut être détendu, être transformé en biométhane par séparation du méthane et du dioxyde de carbone, puis renvoyé dans l'échangeur de chaleur. La figure 3 illustre ce renvoi.

## Revendications

1. Procédé de production de biométhane à partir d'un flux de biogaz comprenant du méthane, du dioxyde de carbone et au moins une impureté choisie parmi l'ammoniac, les composés organiques volatils, l'eau, les impuretés soufrées (H₂S) et les siloxanes, comprenant :
- une première étape de séchage du flux de biogaz,
- une deuxième étape d'élimination au moins partiel de ladite impureté contenue dans le flux de biogaz séché par solidification et retrait de l'impureté ; et
- une troisième étape de séparation du méthane et du dioxyde de carbone contenu dans le biogaz issu de la deuxième étape de manière à produire un flux de biométhane et un flux de CO₂ ;
avec la deuxième étape d'élimination au moins partiel de ladite impureté comprenant :
- une première sous-étape de compression du flux de biogaz séché à une pression supérieure à 5 bar, de préférence 10 bar,
- une deuxième sous-étape de solidification de ladite impureté par refroidissement du flux de biogaz comprimé à une température inférieure à -70°C ; et
- une troisième sous-étape de dépôt et de retrait de ladite impureté.

2. Procédé selon la revendication 1, **caractérisé en ce qu'**à la deuxième sous-étape le flux de biogaz est refroidi par un flux de refroidissement, de préférence un flux de CO₂, produit de préférence lors de la troisième étape.

3. Procédé selon l'une des revendications 1 ou 2, **caractérisé en ce que** dans la troisième sous-étape ladite impureté est retirée par sublimation de ladite impureté et balayage d'un flux gazeux de balayage.

4. Procédé selon la revendication 3, **caractérisé en ce que** la sublimation est effectuée à une température supérieure à la température de sublimation et une pression inférieure à 2 bar.

5. Procédé selon l'une des revendications 3 ou 4, **caractérisé en ce qu'**à la deuxième sous-étape le flux de biogaz est refroidi par un flux de refroidissement et à la troisième sous-étape le flux de refroidissement issu de la deuxième sous-étape est utilisé comme flux gazeux de balayage.

6. Procédé selon la revendication 5, **caractérisé en ce que** la deuxième sous-étape et la troisième sous-étape sont réalisées simultanément en une seule et même sous-étape.

7. Procédé selon l'une des revendications 3 à 6, **caractérisé en ce que** le flux gazeux de balayage est un flux de CO₂ ou d'azote.

8. Procédé selon la revendication 7, **caractérisé en ce que** le flux gazeux de balayage est issu d'une capacité de stockage.

9. Procédé selon la revendication 7, **caractérisé en ce que** le flux gazeux de balayage est le recycle du flux de CO₂ produit lors troisième étape de séparation du méthane et du dioxyde de carbone contenu dans le biogaz.

10. Procédé selon l'une des revendications 1 à 9, **caractérisée en ce que** la troisième étape de séparation du méthane et du dioxyde de carbone contenu dans le biogaz est effectuée par lavage à l'eau ou aux amines, adsorption, solidification du dioxyde de carbone ou séparation membranaire.

11. Procédé selon l'une des revendications 1 à 10, **caractérisée en ce que** la première étape de séchage du flux de biogaz est effectuée par refroidissement et condensation puis adsorption.

12. Procédé selon l'une des revendications 1 à 11, **caractérisée en ce que** la deuxième étape d'élimination au moins partiel de ladite impureté met en œuvre un échangeur de chaleur.

13. Procédé selon l'une des revendications 1 à 9, **caractérisé en ce que** la première sous-étape de compression du flux de biogaz est effectuée à l'aide d'un compresseur.

14. Procédé selon l'une des revendications 1 à 9, **caractérisé en ce que** :
- la deuxième étape d'élimination au moins partiel de ladite impureté met en œuvre un échangeur de chaleur,
- à la deuxième sous-étape le flux de biogaz est refroidi par un flux de refroidissement avec le flux de biogaz passant à travers un premier canal de l'échangeur de chaleur et le flux de refroidissement passant à travers un deuxième canal de l'échangeur de chaleur, et
- à la troisième sous-étape ladite impureté est retirée par sublimation de ladite impureté et balayage d'un flux gazeux de balayage avec le flux gazeux de balayage passant dans le premier canal de l'échangeur de chaleur ;
avec le flux de biogaz passant dans le deuxième canal pendant la troisième sous-étape.
